(19) Europäisches Patentamt
European Patent Office
Office européen des brevets

(11) Veröffentlichungsnummer: **0 015 565**
**B1**

# (12) EUROPÄISCHE PATENTSCHRIFT

(45) Veröffentlichungstag der Patentschrift: **02.06.82**

(21) Anmeldenummer: **80101157.8**

(22) Anmeldetag: **07.03.80**

(51) Int. Cl.³: **C 07 C 47/22,** C 07 C 45/35, B 01 J 35/02, B 01 J 37/02 // (B01J37/02, 23/88)

(54) **Verfahren zur Herstellung von 3 bis 4 C-Atome enthaltenden alpha,beta-olefinisch ungesättigten Aldehyden.**

(30) Priorität: **12.03.79 DE 2909597**

(43) Veröffentlichungstag der Anmeldung: **17.09.80 Patentblatt 80/19**

(45) Bekanntmachung des Hinweises auf die Patenterteilung: **02.06.82 Patentblatt 82/22**

(84) Benannte Vertragsstaaten: **BE DE FR**

(56) Entgegenhaltungen:
**DE-A-2 741 132**
**DE-B-2 135 620**
**US-A-4 077 912**

(73) Patentinhaber: **BASF Aktiengesellschaft, Carl-Bosch-Strasse 38, D-6700 Ludwigshafen (DE)**

(72) Erfinder: **Engelbach, Heinz, Dr., Kropfsburgstrasse 24, D-6703 Limburgerhof (DE)**
Erfinder: **Krabetz, Richard, Dr., Unterer Waldweg 8, D-6719 Kirchheim (DE)**
Erfinder: **Duembgen, Gerd, Dr., Sudetenstrasse 4, D-6701 Dannstadt-Schauernheim 1 (DE)**
Erfinder: **Willersinn, Carl-Heinz, Dr., Erich-Kaestner-Strasse 22, D-6700 Ludwigshafen (DE)**
Erfinder: **Beitelschmidt, Walter, Dr., Berner Weg 34, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 015 565 B1

BUNDESDRUCKEREI BERLIN

# Verfahren zur Herstellung von 3 bis 4 C-Atome enthaltenden $\alpha,\beta$-olefinisch ungesättigten Aldehyden

Diese Erfindung betrifft ein Verfahren zur Herstellung von 3 bis 4 C-Atome enthaltenden $\alpha,\beta$-olefinisch ungesättigten Aldehyden, d. h. von Acrolein bzw. Methacrolein durch katalytische Gasphasenoxidation der entsprechenden Olefine unter Verwendung eines auf verbesserte Weise hergestellten Schalenkatalysators, dessen katalytisch aktive Masse an sich bekannt ist.

Es ist bekannt, Propylen (oder Isobutylen) in der Gasphase mit molekularem Sauerstoff an Katalysatoren im wesentlichen zu Acrolein (bzw. Methacrolein) zu oxidieren, die neben überwiegenden Mengen Molybdän, Eisen, Nickel und/oder Kobalt, Wismut, Phosphor, gegebenenfalls Silicium und gegebenenfalls in kleinen Mengen Alkali und/oder Erdalkalimetalle und/oder seltene Erdmetalle enthalten. Derartige Katalysatoren werden im allgemeinen in Festbett-Reaktoren mit vielen Rohren, im allgemeinen in Tablettenform, eingesetzt, wobei die katalytisch aktive Masse meist mit einem Trägerstoff homogen vermischt wird. Bei dieser Form des Einsatzes der Katalysatoren treten vor allem bei großen Katalysatorbelastungen Probleme hinsichtlich der Abführung der Reaktionswärme auf, wodurch im allgemeinen die Selektivität und Ausbeute beeinträchtigt wird.

Es ist auch aus der GB-PS 1 529 384 und der US-PS 4 077 912 (Beispiel 6) bekannt, Katalysatoren der genannten Art zusammen mit solchen Trägerkatalysatoren bei der Gasphasenoxidation von Propylen (und gegebenenfalls Isobutylen) einzusetzen, bei denen die katalytisch aktive Masse als Schale auf einen Trägerkern aufgebracht ist. Bei der Herstellung dieser Schalenkatalysatoren wird die pulverförmig aktive Masse auf die Trägerteilchen, z. B. Kugeln aus $\alpha$-$Al_2O_3$ eines Durchmessers von 0,32 cm aufgebracht, indem man die porösen Trägerkugeln teilweise mit Wasser sättigt und dann unter rollender Bewegung mit dem pulverförmig katalytisch aktiven Material beschichtet und anschließend calciniert. Hierbei kann eine Propylen-Umwandlung von 95 Prozent bei einfachem Durchgang und eine hohe Selektivität der Acroleinbildung bei einer Temperatur von 355°C und Raumgeschwindigkeiten von 1300 bis 1700 $h^{-1}$ erzielt werden während bei einer entsprechenden alleinigen Verwendung des tablettenförmigen Katalysators keine stabile Reaktion eingestellt werden konnte. Dieses bekannte Verfahren hat den Nachteil, daß wegen der relativ geringen katalytischen Aktivität des Schalenkatalysators, hergestellt nach dem bekannten Verfahren, dieser nur in Kombination mit dem tablettierten Katalysator und bei relativ niedrigen Raumbelastungen unter 1800 $h^{-1}$ einsetzbar ist. Bei Badtemperaturen über 350°C werden auch Maßnahmen zur Verhinderung der Nachverbrennung im Raum nach den Katalysatoren weniger wirksam und weniger wirtschaftlich.

Aus der DE-AS 2 741 132 sind zudem Träger-Katalysatoren für die Gasphasenoxidation von Propen zu Acrolein bekannt, die eine aktive Phase der Formel

$$Bi_2Mo_2Fe_2O_{12}$$

und ggf. Phasen der allgemeinen Formel

$$CoMoO_4,\ Fe_2(MoO_4)_3 \text{ und } Bi_2(MoO_4)_3$$

aufweisen, die zweimal bei Temperaturen zwischen 450 bis 500°C geglüht sind. Die aktiven Katalysatormassen können auf Träger, z. B. auf »rauhe« Kugeln durch Eindampfen ihrer wäßrigen Suspensionen, die die Träger und ein Bindemittel (Glukose) enthalten, aufgebracht sein. Die Aktivität und Selektivität dieser Katalysatoren läßt jedoch zu wünschen übrig.

**Es wurde nun gefunden, daß man Acrolein bzw. Methacrolein durch Oxidation von Propylen bzw. Isobutylen in Sauerstoff enthaltenden Gasgemischen unter an sich üblichen Bedingungen an Trägerkatalysatoren mit einer festhaftenden 150 bis 1500 µm dicken Schale, die calciniertes katalytisch aktives Material der Zusammensetzung**

$$Mo_{12}Me^1_aMe^2_bMe^3_cMe^4_dMe^5_eO_x$$

**enthält, in der**
**$Me^1$ für Bi und/oder Sb,**
**$Me^2$ für Ni, Co, Fe, und/oder Cu und gegebenenfalls zusätzlich Zn und/oder Mg,**
**$Me_3$ für K, Rb, Cs, und/oder Tl,**
**$Me^4$ für P und/oder B,**
**$Me^5$ für Sn, W, Nb, Ta, Cr, Pb, In und/oder Na,**
**a für 0,1 bis 13,**
**b für 0,5 bis 15,**
**c für 0,01 bis 4,**
**d für 0 bis 2,**
**e für 0 bis 3 und**
**X für die Zahl der zur Absättigung der Valenzen der anderen Bestandteile erforderlichen Sauerstoffatome**

stehen und einem Träger einer Oberfläche von unter 15 $m^2/g$ und eines Durchmessers über 100 µm mit besonderem Vorteil herstellen kann, indem man einen Schalenkatalysator einsetzt, bei dessen Herstellung auf die gegebenenfalls zunächst mit bis maximal 95% ihres Wasseraufnahmewertes Wasser befeuchteten stark bewegten Träger kontinuierlich mit jeweils konstanter Dosiergeschwindigkeit 1 bis 40 g/min/l Träger an calciniertem katalytisch aktivem Material einer Teilchengröße von 0,1 bis 300 µm und gleichzeitig aber räumlich getrennt davon Wasser aufgegeben wurden, wobei das Verhältnis von katalytisch aktivem Material zu aufgegebenem Wasser 1 : 1 bis 8 : 1 betrug und der Wassergehalt der aufwachsenden Schale unter dem maximalen Sättigungsgrad des katalytisch aktiven Materials lag.

Im Falle der Oxidation von Propylen zu überwiegend Acrolein besitzt das katalytisch aktive Material vorzugsweise die Zusammensetzung

$$Mo_{12}Bi_{0,1-4}Fe_{0,5-6}Me^2_bMe^3_cMe^4_dMe^5_eO_x$$

in der
$Me^2$ für Nickel und/oder Kobalt und gegebenenfalls zusätzlich Zn und/oder Magnesium,
$Me^3$ für mindestens ein Element aus der Gruppe K, Rb, Cs und/oder Tl,
$Me^4$ für P,
$Me^5$ für In und/oder Na und
b für 2 bis 12, vorzugsweise 4 bis 10, wobei b bei
$Me^2$ = Ni + Zn zusammen für 8 bis 9 mit b für
Ni = 5,5 bis 8,5,
c für 0,01 bis 0,1, vorzugsweise 0,03 bis 0,09,
d für 0 bis 1, vorzugsweise 0,01 bis 0,2,
e für 0 bis 0,5, vorzugsweise 0,01 bis 0,2 und
x für die Zahl der zur Absättigung der Valenzen der anderen Bestandteile erforderlichen Sauerstoffatome stehen.

Das katalytisch aktive Material, das für die Katalysatorherstellung eingesetzt wird, kann in an sich üblicher und bekannter Weise durch Vermischen wäßriger Lösungen von vorzugsweise leicht zersetzlichen Salzen der Komponenten, Eindampfen des Gemischs und/oder Sprühtrocknen, gegebenenfalls Verdichten und gegebenenfalls mehrstufigem Calcinieren meist bei Temperaturen von 250 bis 700°C, vorzugsweise in 2 Stufen bei Temperaturen von 250 bis 400°C und 480 bis 660°C sowie Vermahlen auf Teilchengrößen, die im allgemeinen 0,1 bis 300, vorzugsweise 0,2 bis 150, insbesondere 0,5 bis 50 µm betragen, hergestellt sein.

Das pulverförmige calcinierte katalytisch aktive Material wird dann auf die stark bewegten, mit Wasser vorbefeuchteten Träger unter gleichzeitigem räumlich getrennten Aufbringen, meist Aufsprühen, von Wasser schalenförmig auf die Träger mit jeweils konstanter Dosiergeschwindigkeit für Katalysatorpulver und Wasser aufgebracht. Dabei beträgt die Menge des je Minute und Liter Träger aufgebrachten calcinierten katalytisch aktiven Materials 1 bis 40, vorzugsweise 2 bis 25 g. Es kann beispielsweise auf die auf einem Drehteller oder in einer Dragiertrommel in starker Bewegung gehaltenen Träger mittels einer Dosierbandwaage und/oder Dosierschnecke mit Schüttelrinne und Verteilervorrichtung aufgebracht werden, wobei gleichzeitig, aber räumlich getrennt davon, das Wasser aufgesprüht wird. Hierbei soll das pulverförmige katalytisch aktive Material außerhalb des Sprühkegels des Wassers auf die rollenden Träger auftreffen und das Gewichtsverhältnis von Katalysatorpulver zu Wasser vorzugsweise im Bereich von 2 : 1 bis 5 : 1 liegen. Die Vorbefeuchtung von porösem Träger mit Wasser soll mindestens 0,1% seines Gewichts betragen und im allgemeinen 95% seines Wasseraufnahmewertes nicht übersteigen. Unter Wasseraufnahmewert wird die Menge Wasser in g verstanden, die 100 g des Trägers in der Form, in der er bei der Herstellung der Katalysatoren eingesetzt wird, maximal aufnehmen. Das Ausmaß der Vorbefeuchtung hängt von der Porosität des Trägers ab und soll bei bevorzugter Porositäten unter 5% nur etwa 0,1 bis 2 Gewichtsprozent, bezogen auf den Träger, betragen und 95% des Wasseraufnahmewertes nicht übersteigen. Bei Porositäten über 5% ist ein Vorbefeuchtungsgrad von 5 bis 30, vorzugsweise von 10 bis 25% des Wasseraufnahmewertes des Trägers von Vorteil. Unporöse Träger brauchen nicht vorbefeuchtet zu werden. Die Träger, die aus den üblichen Materialien bestehen können, beispielsweise aus α-Aluminiumoxid, Siliciumdioxid, Silikaten, wie Magnesiumsilikat und Magnesiumaluminiumsilikat sowie Siliciumcarbid, sollen vorzugsweise eine Oberfläche von weniger als 15 $m^2/g$, insbesondere weniger als 5 $m^2/g$, aufweisen. Die Träger können geformt oder ungeformt sein, bevorzugt werden Kugeln eines Durchmessers von 0,5 bis 6 mm, vorzugsweise von 1 bis 5 mm. Die Träger besitzen vorteilhaft eine Porosität unter 5% und eine natürliche oder künstliche Oberflächenrauhigkeit. Die Menge an katalytisch aktivem Material liegt bei den für das neue Verfahren eingesetzten Schalenkatalysatoren im Bereich von 50 und 250 Gew.-%, vorzugsweise von 55 und 200 Gew.-%, insbesondere bei Katalysatoren für die Isobutenoxidation von 100 bis 180 Gew.-%, bezogen auf das Trägergewicht, und die Schichtdicke der Schalen soll im allgemeinen 150 bis 1500 µm, vorzugsweise 300 bis 1200 µm betragen. Bei der Erfindungsgemäßen Beschichtungsmethode

beträgt der Befeuchtungsgrad der auf die Träger aufwachsenden Schalen während der Beschichtung im allgemeinen 40 bis 95, vorzugsweise 50 bis 90% des maximalen Sättigungsgrades. Der maximale Sättigungsgrad der Schale entspricht einem Feuchtigkeitsgehalt, bei dem die teilweise beschichteten Träger zu agglomerieren beginnen, d. h. nicht mehr als isolierte Teilchen durch den Beschichtungsapparat rollen. Näherungsweise ist unter dem maximalen Sättigungsgrad der Schale die Wassermenge in g zu verstehen, die von 100 g des gerührten Katalysatorpulvers aufgenommen wird, bis die bei tropfenweiser Wasserzugabe entstehenden Agglomerate oberflächlich feucht und klebrig werden. Die Schalenkatalysatoren werden vor ihrem Einsatz bei der Oxidation der Olefine auf einen Wassergehalt von 0,1 bis 2% ihres Gewichts getrocknet, wobei im allgemeinen Temperaturen unter 150° C angewandt werden. Eine Calcinierung des Katalysators bei erhöhten Temperaturen, z. B. bei 400 bis 700° C, ist zwar möglich, jedoch im allgemeinen nicht erforderlich.

Das neue Oxidationsverfahren kann in den üblichen Röhrenreaktoren, in denen die Reaktionsrohre im allgemeinen einen Durchmesser von 15 bis 25 mm haben und von geschmolzenen Salzmischungen als Wärmeübertragungsmittel umströmt werden durchgeführt werden. Der Betriebsdruck liegt bei dem neuen Verfahren meist im üblichen Bereich von 1,2 bis 3 bar. Das Reaktionsgas am Eingang des Reaktors weist z. B. bei der Propylenoxidation im allgemeinen die übliche prozentuale Zusammensetzung von 3 bis 8 Volumenprozent Propylen, 6 bis 12 Volumenprozent Sauerstoff und gegebenenfalls 80 bis 91 Volumenprozent Inertgase, z. B. Wasserdampf, Stickstoff, Kohlenoxide, auf.

Die Salzbadtemperaturen liegen bei der Propylenoxidation meist im Bereich von 300 bis 350° C, vorzugsweise von 310 bis 340° C, bei der Isobutenoxidation im Bereich von 350 und 450° C. Beim Einsatz der erfindungsgemäß hergestellten Schalenkatalysatoren ist die Mitverwendung üblicher nicht schalenförmigen Trägerkatalysatoren, wie dies aus der DE-OS 2 611 249 bekannt ist, zwar möglich, aber nicht erforderlich. Es ist jedoch vorteilhaft, Schalenkatalysatoren mit unterschiedlichem Gehalt an aktiver Masse und damit unterschiedlicher Aktivität in einem Reaktionsrohr derart anzuordnen, daß die Aktivität in Strömungsrichtung kontinuierlich oder stufenweise ansteigt. Das neue Verfahren ermöglicht besonders hohe Katalysatorbelastungen und damit hohe Raum-Zeit-Ausbeuten, wobei die Bildung unerwünschter Nebenprodukte, überraschenderweise im Vergleich zu den herkömmlichen Verfahren in manchen Fällen zurückgedrängt wird. Bei dem neuen Verfahren kann in an sich bekannter Weise das Reaktionsgemisch unmittelbar, gegebenenfalls unter Zusatz von weiterem Sauerstoff und/oder Verdünnungsmitteln in einem weiteren, einen hierfür spezifischen, gegebenenfalls bekannten Katalysator enthaltenden Reaktor im wesentlichen zu Acrylsäure (bzw. Methacrylsäure) weiter oxidiert werden und das dabei erhaltene Restgas nach Abtrennung der Reaktions- und Nebenprodukte sowie gegebenenfalls von Wasser als Kreisgas an den Eingang des Propylen(isobuten)-Oxidations-Reaktors zurückgeführt und dort mit Frischgas vermischt werden.

Die in den folgenden Beispielen angegebenen maximalen Sättigungsgrade für das pulverförmige calcinierte katalytisch aktive Material wurden wie folgt bestimmt:

Auf 100 g des in einer Porzellanschale gerührten Katalysator-Pulvers wird innerhalb von 5 bis 10 Minuten tropfenweise so lange Wasser zugegeben, bis die sich ausbildenden Agglomerate die pulverförmigen Anteile vollständig aufgenommen haben und oberflächlich feucht und klebrig werden. Der durch Rückwiegung bestimmte Wasserverbrauch, bezogen auf das Gewicht des trockenen Pulvers, ist der maximale Sättigungsgrad.

Der in den folgenden Beispielen angegebene Wasseraufnahmewert des Trägers wird wie folgt bestimmt:

100 g des Trägers in der bei der Herstellung des Katalysators vorliegenden Form werden unter Wasser gesättigt, in einen schwach angefeuchteten Papierfilter eingefüllt und das überschüssige Wasser 1/4 Stunde abtropfen lassen. Die Gewichtszunahme des Trägers, bezogen auf das Trockengewicht des Trägers, ist der Wasseraufnahmewert des Trägers.

Die in den folgenden Beispielen angegebenen Prozente beziehen sich auf das Gewicht.

## Beispiel 1

### (a) Herstellung des Katalysators

Eine katalytisch aktives Material der Zusammensetzung

$$Mo_{12}Ni_{8,5}Fe_2Bi_1P_{0,06}K_{0,06}O_x + 10\ SiO_2$$

wird wie folgt hergestellt:

Man mischt in der angegebenen Reihenfolge 561 g Wismutnitratlösung (11% Bi, 5% freie Salpetersäure), 1117 g Nickelnitratlösung (13,2% Nickel), 239 g Eisennitrat in 1240 g Wasser. Zu dieser Nitratlösung gibt man eine Lösung von 625 g Ammoniumheptamolybdat und 2,3 g 75%ige Phosphorsäure sowie 452 g 25%ige Ammoniaklösung in 3320 g Wasser. Dann fügt man noch 354 g 50%iges wäßriges Kieselsol zu und trocknet die erhaltene Suspension unter Versprühen.

500 g des sprühgetrockneten Materials werden unter Zusatz von 62 g Wasser und 0,34 g 48%iger Kalilauge 1 1/2 Stunden geknetet und zu Strängen eines Durchmessers von 4,5 mm geformt. Die Stränge werden 24 Stunden bei 120° C getrocknet, sodann 2 Stunden bei 360° C unter Luftzutritt calciniert und anschließend auf eine Teilchengröße unter 300 µm gemahlen. Der maximale Sättigungsgrad des calcinierten katalytisch aktiven Materials beträgt 48%. Dieses Pulver wird mit 6 Gew.-% Pharmacoat ® (Methylcellulose der Firma gemischt.

2600 g des Pulvers werden mit einer Dosiergeschwindigkeit von 17,4 g/Liter Träger je Minute auf 2500 g (1,78 Liter) mit 32 g Wasser vorbefeuchteten Träger gegeben und gleichzeitig kontinuierlich und räumlich getrennt, 850 g Wasser mit einer Geschwindigkeit von 5,9 g je Liter Träger je Minute durch eine Düse zugesprüht. Als Träger werden handelsübliche Magnesiumsilikat-Kugeln eines Durchmessers von 1,5 bis 2,5 mm verwendet, deren Porosität etwa 1% beträgt. Die Beschichtung wird auf einem Drehteller von 50 cm Durchmesser bei 14 Umdrehungen je Minute durchgeführt. Der mittlere Befeuchtungsgrad der aufwachsenden Schale beträgt 71% des maximalen Sättigungsgrades. Nach beendeter Beschichtung wird der erhaltene Schalenkatalysator 16 Stunden bei 80° C getrocknet und anschließend 1 1/2 Stunden bei 600° C in Luftstrom calciniert.

(b) Oxidation von Propylen zu Acrolein

800 $cm^3$ des unter (a) erhaltenen Schalenkatalysators werden in einem Stahlrohr von 3,6 m Länge und einer lichten Weite von 21 mm auf 342 bis 343° C erhitzt. Über den erhitzten Katalysator wird ein Gemisch von stündlich 80 Normalliter Propylen, 800 Normalliter Luft und 800 Normalliter Stickstoff geleitet. Man erhält einen Propylenumsatz von 95,3 Molprozent und eine Ausbeute an Acrolein von 83,5 und an Acrylsäure von 6 Molprozent. Die Selektivität für Acrolein und Acrylsäure beträgt somit 93,9%.

Bei einer Reaktionstemperatur von 332° C wird unter sonst gleichen Bedingungen ein Propylenumsatz von 87,8 Molprozent, eine Ausbeute an Acrolein und an Acrylsäure von 83,7 Molprozent und eine Selektivität von 95,3 Molprozent erreicht.

Bei einem weiteren Versuch, bei dem die Belastung auf 96 Normalliter Propylen, 960 Normalliter Luft und 960 Normalliter Stickstoff erhöht ist, wurde bei einer Badtemperatur von 340° C unter sonst gleichen Bedingungen ein Umsatz von 95 Mol-%, eine Ausbeute von Acrolein und an Acrylsäure von 88,7 Mol-% und eine Selektivität von 93,4 Mol-% erreicht.

(c) Weitere Schalenkatalysatoren und ihre Verwendung für die Propylenoxidation

Wie unter (a) angegeben, werden katalytisch aktive Materialien der Zusammensetzung

$Mo_{12}Ni_{6,5}Zn_2Fe_2Bi_1P_{0,065}K_{0,06}O_x + 10\ SiO_2$
$Mo_{12}Ni_{6,5}Zn_2Fe_2Bi_1P_{0,05}Rb_{0,06} + 10\ SiO_2$ und
$Mo_{12}Ni_{6,5}Zn_2Fe_2Bi_1P_{0,06}In_{0,065}K_{0,06}Na_{0,2} + 10\ SiO_2$

hergestellt und in Mengen von 140 g je 100 g Träger auf die Magnesiumsilikatkugeln aufgetragen. Es werden sehr abriebfeste Katalysatoren erhalten, die z. B. unter den Bedingungen von (b) sehr gut für die Oxidation von Propylen zu Acrolein geeignet sind.

## Beispiel 2

(a) Herstellung des Katalysators

Es wurde eine katalytisch aktives Material der Zusammensetzung

$Mo_{12}Bi_1Fe_3Ni_1Co_7B_2Sb_{0,1}K_{0,14}O_{56,7}$

nach den Angaben in Beispiel 1 der GB-PS 1 491 750 hergestellt. Das erhaltene katalytisch aktive Material wird auf eine Teilchengröße unter 30 µm gemahlen. Der maximale Sättigungsgrad des Katalysatorpulvers beträgt 33,4%.

156 g des pulverförmigen katalytisch aktiven Materials werden auf 100 g (entsprechend 76 $cm^3$) unporöse Steatitkugeln von 3 mm Durchmesser, dit 1 g Wasser vorbefeuchtet sind auf einem Drehteller von 30 cm Durchmesser kontinuierlich mit jeweils konstanter Dosiergeschwindigkeit von 17,3 g je Liter Träger je Minute und getrennt davon gleichzeitig mit 6,9 g je Liter Träger je Minute Wasser bei 20 bis 25° C besprüht. Beim Versprühen des Wassers werden dabei 12,5 Normalliter je Minute Luft eingesetzt. Die Drehzahl des Tellers beträgt 35 Umdrehungen je Minute. Unter den genannten Bedingungen beträgt der Wassergehalt der aufwachsenden Schale 67% des maximalen Sättigungsgrades des katalytisch aktiven Katalysatorpulvers. Die erhaltene Schalenkatalysator hat im Mittel einen Durchmesser von 4,7 mm, entsprechend einer mittleren Schichtdicke der Schale von 0,85 mm. Die Abriebfestigkeit des Katalysators ist sehr gut.

(b) Oxidation von Isobuten zu Methacrolein

In einem Stahlrohr einer lichten Weite von 15 mm werden 43 $cm^3$ des Schalenkatalysators auf 376° C geheizt und darüber ein Gemisch von je Stunde 3 Normalliter Isobutylen, 37,2 Normalliter Luft und 24 Normalliter Wasserdampf geleitet. Der dabei erzielte Umsatz an Isobuten beträgt 94 Molprozent, die Ausbeute an Methacrolein 80 Molprozent und die Ausbeute an Methacrylsäure 1 Molprozent, die Gesamtselektivität somit 87 Molprozent.

(c) Weitere Schalenkatalysatoren und ihre Verwendung für die Oxidation von Isobutylen

Es werden katalytisch aktive Materialien nach den Angaben der Beispiele 2, 6, 9 und 18 der GB-PS 14 90 683 hergestellt und in Mengen von 150 g je 100 g Träger wie unter (a) angegeben, auf die Magnesiumsilikatkugeln aufgetragen. Die Schalenkatalysatoren sind sehr gut für die Oxidation von Isobuten zu Methacrolein geeignet.

**Patentansprüche**

1. Verfahren zur Herstellung von Acrolein bzw. Methacrolein durch Oxidation von Propylen bzw. Isobutylen in Sauerstoff enthaltenden Gasgemischen unter an sich üblichen Bedingungen an Trägerkatalysatoren mit einer festhaftenden 150 bis 1500 µm dicken Schale, die calciniertes katalytisch aktives Material der Zusammensetzung

$$Mo_{12}Me^1_aMe^2_bMe^3_cMe^4_dMe^5_eO_x$$

enthält, in der
$Me^1$ für Bi und/oder Sb
$Me^2$ Ni, Co, Fe und/oder Cu und gegebenenfalls zusätzlich Zn und/oder Mg
$Me^3$ für K, Rb, Cs und/oder Tl
$Me^4$ für P und/oder B,
$Me^5$ für Sn, W, Nb, Ta, Cr, Pb, In und/oder Na
a für 0,1 bis 13
b für 0,5 bis 15
c für 0,01 bis 4
d für 0 bis 2
e für 0 bis 3
x für die Zahl der zur Absättigung der Valzenzen der anderen Bestandteile erforderlichen Sauerstoffatome

stehen und einem Träger einer Oberfläche unter 15 $m^2/g$ und einem Durchmesser von über 100 µm, dadurch gekennzeichnet, daß Schalenkatalysatoren eingesetzt werden, bei deren Herstellung auf die gegebenenfalls zunächst mit bis maximal 95% ihres Wasseraufnahmewertes Wasser befeuchteten, stark bewegten Träger bei einer Temperatur unter 100°C kontinuierlich mit jeweils konstanter Dosiergeschwindigkeit 1 bis 40 g/min/l Träger an calciniertem katalytisch aktivem Material einer Teilchengröße von 0,1 bis 300 µm und gleichzeitig aber räumlich getrennt davon Wasser aufgegeben wurde, wobei das Verhältnis von katalytisch aktivem Material zu aufgegebenem Wasser 1 : 1 bis 8 : 1 betrug und der Wassergehalt der aufwachsenden Schale unter dem maximalen Sättigungsgrad des katalytisch aktiven Materials lag.

2. Verfahren nach Anspruch 1, dadurch gekennzeichnet, daß der Träger des Schalenkatalysators eine Porosität unter 5% hat und vor dem Aufgeben des katalytisch aktiven Materials mit 0,1 bis 2% seines Gewichtes Wasser befeuchtet wurde.

3. Verfahren nach Anspruch 1, dadurch gekennzeichnet, daß der Träger des Schalenkatalysators eine Porosität über 5% hat und vor dem Aufgeben des katalytisch aktiven Materials mit 10 bis 30% seines Wasseraufnahmewertes Wasser befeuchtet wurde.

4. Verfahren nach Anspruch 1, dadurch gekennzeichnet, daß die katalytisch aktive Masse die Zusammensetzung

$$Mo_{12}Bi_{0,1-4}Fe_{0,5-6}Me^2_{2-12}Me^3_{0,01-0,1}Me^4_{0-1}Me^5_{0-0,5}O_x$$

$Me^2$ für Ni und/oder Co und gegebenenfalls zusätzlich für Zn und/oder Mg,
$Me^3$ für mindestens ein Element aus der Gruppe K, Rb, Cs und/oder Tl,
$Me^4$ für P,
$Me^5$ für In und/oder Na und
x für die Zahl der zur Absättigung der Valenzen der anderen Bestandteile erforderlichen Sauerstoffatome

stehen.

**Claims**

1. A process for the preparation of acrolein or methacrolein by oxidation of propylene or isobutylene with an oxygen-containing gas mixture under conventional conditions over a supported catalyst possessing a firmly adhering coating, from 150 to 1,500 µm thick, which contains calcined catalytic material of the composition

$$Mo_{12}Me^1_aMe^2_bMe^3_cMe^4_dMe^5_eO_x$$

where
$Me^1$ is Bi and/or Sb,
$Me^2$ is Ni, Co, Fe and/or Cu, with or without Zn and/or Mg,
$Me^3$ is K, Rb, Cs and/or Tl,
$Me^4$ is P and/or B,
$Me^5$ is Sn, W, Nb, Ta, Cr, Pb, In and/or Na,
a is from 0.1 to 13,
b is from 0.5 to 15,
c is from 0.01 to 4,
d is from 0 to 2,
e is from 0 to 3, and
x is the number of oxygen atoms required to saturate the valencies of the other constituents,
and a carrier having a surface area of less than 15 $m^2/g$ and a diameter of more than 100 µm, characterized in that coated catalysts are employed in the preparation fo which calcined catalytic material having a particle size f from 0.1 to 300 µm, in an amount of from 1 to 40 g/min/liter of carrier, and water were continuously applied simultaneously but at spatially separate points, each at a constant speed, to the vigorously agitated carrier which may or may not have been premoistened with water in an amount of up to a maximum of 95% of the water absorbency of the particles, the weight ratio of catalytic material to water being from 1 : 1 to 8 : 1, and the water content of the coating which forms being less than the maximum degree of saturation of the catalytic material.

2. A process as claimed in claim 1, characterized in that the carrier of the coated catalyst has a porosity of less than 5% and has been moistened with from 0.1 to 2%, of its weight, of water before being coated with the catalytic material.

3. A process as claimed in claim 1, characterized in that the carrier of the coated catalyst has a porosity of more than 5% and has been moistened with from 10 to 30%, of its water absorbency, of water before being coated with the catalytic material.

4. A process as claimed in claim 1, characterized in that the catalytic material has the composition

$$Mo_{12}Bi_{0.1-4}Fe_{0.5-6}Me^2_{2-12}Me^3_{0.01-0.1}Me^4_{0-1}Me^5_{0-0.5}O_x$$

where
$Me^2$ is Ni and/or Co, with or without Zn and/or Mg,
$Me^3$ is at least one element from the group consisting of K, Rb, Cs and/or Tl,
$Me^4$ is P,
$Me^5$ is In and/or Na and
x is the number of oxygen atoms required to saturate the valencies of the other constituents.

**Revendications**

1. Procédé de préparation de l'acroléine et(ou) de la méthacroléine par oxydation du propylène ou de l'isobutylène dans des mélanges de gaz comprenant l'oxygène, dans des conditions opératoires connues en soi, sur des catalyseurs sur support, constitués d'un support d'une surface inférieure à 15 $m^2/g$ en particules d'un diamètre supérieur à 100 µm, entourées d'une enveloppe fortement adhérente d'une épiasseur de 150 à 1500 µm, contenant une matière à acticité catalytique calcinée de la composition

$$Mo_{12}Me^1_aMe^2_bMe^3_cMe^4_dMe^5_eO_x$$

dans laquelle
$Me^1$ = Bi et(ou) Sb
$Me^2$ = Ni, Co, Fe et(ou) Cu et éventuellement en outre Zn et(ou) Mg
$Me^3$ = K, Rb, Cs et(ou) Tl
$M^4$ = P et(ou) B
$Me^5$ = Sn, W, Nb, Ta, Cr, Pb, In et(ou) Na
a vaut 0,1 à 13

b vaut 0,5 à 15
c vaut 0,01 à 4
d vaut 0 à 2
e vaut 0 à 3 et
x représente le nombre d'atomes d'oxygène nécessaires pour la saturation des valences des autres composants,

caractérisé en ce que l'on emploie des catalyseurs à particules enrobées, préparés en ajoutant au support, éventuellement mouillé avec de l'eau jusqu'à, au maximum, 95% de son pouvoir d'absorption d'eau, fortement agité, à une température inféreieure à 100° C, de manière continue et sous des débits constants 1 à 40 g, par mn et par 1 de support, de matière à activité catalytique calcinée en particules d'une granulométrie de 0,1 à 300 µm et simultanément, mais en un point éloigné dans l'espace, de l'eau, le rapport entre la matière à activité catalytique et l'eau ajoutée étant compris entre 1 : 1 et 8 : 1 et la teneur en eau de l'enveloppe en voie de formation étant maintenue inférieure au degré de saturation maximal de la matière à activité catalytique.

2. Procédé suivant la revendication 1, caractérisé en ce que le support du catalyseur à particules enrobées possède une porosité inféreure à 5% et a été mouillé avec 0,1 à 2% de son poids d'eau avant l'addition de la matière à activité catalytique.

3. Procédé suivant la revendication 1, caractérisé en ce que le support du catalyseur à particules enrobées possède une porosité supéreure à 5% et a été mouillé avec de l'eau jusqu'à 10 à 30% de son pouvoir d'absorption d'eau avant l'addition de la matière à activité catalytique.

4. Procédé suivant la revendication 1, caractérisé en ce que la matière à activité catalytique possède la composition

$$Mo_{12}Bi_{0,1-4}Fe_{0,5-6}Me^2_{2-12}Me^3_{0,01-0,1}Me^4_{0-1}Me^5_{0-0,5}O_x$$

dans laquelle
$Me^2$ = Ni et(ou) Co et éventuellement en outre Zn et(ou) Mg
$Me^3$ = au moins un élément du groupe K, Rb, Cs et(ou) Tl
$Me^4$ = P
$Me^5$ = In et(ou) Na et
x représente le nombre des atomes d'oxygène nécessaires pour la saturation des valences des autres composants.